(19) Europäisches Patentamt
European Patent Office
Office européen des brevets

(11) **EP 3 550 333 B1**

(12) **FASCICULE DE BREVET EUROPEEN**

(45) Date de publication et mention
de la délivrance du brevet:
**10.03.2021   Bulletin 2021/10**

(51) Int Cl.:
***G01V 11/00*** *(2006.01)*

(21) Numéro de dépôt: **19163140.7**

(22) Date de dépôt: **15.03.2019**

(54) **PROCEDE POUR SIMULER LA QUANTITE ET LA QUALITE DES HYDROCARBURES D'UN BASSIN SEDIMENTAIRE**

SIMULATIONSVERFAHREN DER QUANTITÄT UND QUALITÄT VON KOHLENWASSERSTOFFEN EINES SEDIMENTBECKENS

METHOD FOR SIMULATING THE AMOUNT AND THE QUALITY OF HYDROCARBONS FROM A SEDIMENTARY BASIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **05.04.2018   FR 1852943**

(43) Date de publication de la demande:
**09.10.2019   Bulletin 2019/41**

(73) Titulaire: **IFP Energies nouvelles**
**92852 Rueil-Malmaison Cedex (FR)**

(72) Inventeurs:
• **DUCROS, Mathieu**
**92852 RUEIL-MALMAISON CEDEX (FR)**
• **TRABY, Renaud**
**92852 RUEIL-MALMAISON CEDEX (FR)**

(56) Documents cités:
**EP-A1- 0 691 540        EP-A1- 2 816 377**
**WO-A1-2014/040622     CN-A- 106 837 317**
**US-A1- 2010 228 485**

## Description

La présente invention concerne le domaine de l'exploration et de l'exploitation des gisements pétroliers ou de sites de stockage géologique de gaz.

L'exploration pétrolière consiste à rechercher des gisements d'hydrocarbures au sein d'un bassin sédimentaire. La compréhension des principes de la genèse des hydrocarbures et leurs liens avec l'histoire géologique du sous-sol, a permis la mise au point de méthodes d'évaluation du potentiel pétrolier d'un bassin sédimentaire. La démarche générale de l'évaluation du potentiel pétrolier d'un bassin sédimentaire comporte des allers-retours entre :

- une prédiction du potentiel pétrolier du bassin sédimentaire, réalisée à partir d'informations disponibles concernant le bassin étudié (affleurements, campagnes sismiques, forages par exemple). Cette prédiction vise :

  - à mieux comprendre l'architecture et l'histoire géologique du bassin étudié, notamment à étudier si des processus de maturation et de migration d'hydrocarbures ont pu se mettre en place ;

  - à identifier les zones du sous-sol dans lesquelles ces hydrocarbures ont pu s'accumuler ;

  - à définir quelles zones présentent le meilleur potentiel économique, évalué à partir du volume et de la nature des hydrocarbures probablement piégés (viscosité, taux de mélange avec de l'eau, composition chimique, etc), ainsi que de leur coût d'exploitation (contrôlé par exemple par la profondeur et la pression de fluide).

- des forages d'exploration dans les différentes zones présentant le meilleur potentiel, afin de confirmer ou infirmer le potentiel prédit préalablement, et d'acquérir de nouvelles données pour alimenter de nouvelles études plus précises.

L'exploitation pétrolière d'un gisement consiste, à partir des informations récoltées lors de la phase d'exploration pétrolière, à sélectionner les zones du gisement présentant le meilleur potentiel pétrolier, à définir des schémas d'exploitation optimaux pour ces zones (par exemple à l'aide d'une simulation de réservoir, afin de définir les nombre et positions des puits d'exploitation permettant une récupération d'hydrocarbures optimale), à forer des puits d'exploitation et, de façon générale, à mettre en place les infrastructures de production nécessaires au développement du gisement.

Dans certains bassins sédimentaires, ayant subi une histoire géologique compliquée, faisant interagir de nombreux processus physiques, ou lorsque le volume de données est très important, l'évaluation du potentiel pétrolier d'un bassin sédimentaire requiert de disposer d'outils informatiques permettant la synthèse des données disponibles, et d'outils informatiques permettant la simulation de l'histoire géologique et des multiples processus physiques qui la contrôlent. Il s'agit d'une démarche dite de « modélisation de bassin ». La famille des logiciels dits de modélisation de bassin permettent de simuler en une, deux ou trois dimensions, les processus sédimentaires, tectoniques, thermiques, hydrodynamiques et de chimie organique et inorganique qui interviennent lors de la formation d'un bassin pétrolier. La modélisation de bassin comporte classiquement trois étapes :

- une étape de construction d'une représentation maillée du bassin étudié, connue sous le terme de géo-modélisation. Cette représentation maillée est le plus souvent structurée en couches, c'est-à-dire qu'un groupe de mailles est affecté à chaque couche géologique du bassin modélisé. Puis, chaque maille de cette représentation maillée est remplie par une ou plusieurs propriétés pétrophysiques, telles que la porosité, le faciès (argile, sable, etc) ou encore leur teneur en matière organique au moment de leur sédimentation. La construction de ce modèle se base sur des données acquises au cours de campagnes sismiques, de mesures dans des puits, de carottages, etc.

- une étape de reconstruction structurale de cette représentation maillée, représentant des états antérieurs de l'architecture du bassin. Cette étape peut être réalisée à l'aide d'une méthode dite de "backstripping" (Steckler et al., 1978) ou encore par une méthode dite de restauration structurale (EP 2110686).

- une étape de simulation numérique d'une sélection de phénomènes physiques se déroulant au cours de l'évolution du bassin et contribuant à la formation des pièges pétroliers. Cette étape, connue sous le terme de « simulation de bassin », s'appuie sur une représentation discrétisée de l'espace et du temps. En particulier, une simulation de bassin fournit une cartographie prédictive du sous-sol, indiquant l'emplacement probable des gisements, ainsi que la teneur, la nature et la pression des hydrocarbures qui y sont piégés.

En fournissant des informations quantitatives et fiables, cette démarche intégrée de modélisation de bassin permet d'augmenter le taux de succès lors d'un forage d'un puits d'exploration.

On connait des outils de simulation de bassin qui permettent de simuler numériquement la formation d'un bassin sédimentaire. On citera par exemple l'outil décrit dans le brevet EP2110686 (US8150669) ou les demandes de brevet EP2816377 (US2014/0377872), EP3075947 (US2016/0290107), EP3182176 (US2017/0177764). Ces outils permettent notamment d'estimer l'évolution de la température dans l'ensemble d'un bassin sédimentaire au cours des temps géologiques.

L'essentiel des réserves pétrolières connues correspond à des fluides d'origine organique. La matière organique est très variable en termes de composition élémentaire et de potentiel de génération d'hydrocarbures. Ces variations s'expliquent d'une part par l'origine même de la matière organique, qui peut provenir de micro-organismes marins, d'algues lacustres ou de plantes supérieures par exemple, et d'autre part, de l'état de préservation de cette matière organique qui peut notamment être oxydée ou dégradée par des micro-organismes.

Les principales étapes d'évolution de la matière organique sont très fortement liées à l'augmentation de la température dans le sous-sol, due en particulier à l'enfouissement progressif des sédiments (Vandenbroucke et Largeau, 2007). Pendant l'étape de catagenèse on assiste à la transformation du kérogène en hydrocarbures. Cette transformation de la matière organique est une fonction du temps et de la température (Ungerer 1990 ; McNab et al., 1952 ; Pitt, 1961 ; Philippi, 1965 ; Louis et Tissot, 1967).

De manière à prédire de manière fiable les quantités d'hydrocarbures produites par les roches mères dans les bassins sédimentaires, il est important que ces modèles de bassin simulent, de la manière la plus réaliste possible, la transformation de la matière organique au cours des temps géologiques dans un bassin. Une telle simulation peut être obtenue à partir d'un modèle quantitatif de transformation de la matière organique présente dans la roche mère du bassin étudié, combinée avec la prédiction de l'évolution de la température dans le bassin.

### État de la technique

Les documents suivants seront cités au cours de la description :

- John G.Stainforth, Practical kinetic modeling of petroleum generation and expulsion. Marine and Petroleum Geology Volume 26, Issue 4, April 2009, Pages 552-572
- Pepper and Corvi, 1995, Simple Kinetic models of petroleum formation Marine and Petroleum Geology 12(3):291-319
- Ungerer, 1990,State of the art of research in kinetic modelling of oil formation and expulsion Org. Geochem. Vol. 16, Nos 1-3, pp. 1-25, 1990
- McNab, J.G., Smith, P.V., Jr and Betts, R L., 1952, The évolution of petroleum. Ind. Engin. Chem., 44, 2556
- Pitt, G.J., 1961, The kinetics of the évolution of volatile products form coal. In: 4th International Conférence on Coal Science, Le Touquet, France, 30 May-2 June 1961.-2563.
- Louis, M.C. and Tissot, B. P., 1967, Influence de la température et de la pression sur la formation des hydrocarbures dans les argiles à kérogène. In: Proceedings, 7th World Petroleum Congress, Mexico, Vol. 2, 47-60.
- Philippi, G.T., 1965, On the depth, time and mechanism of petroleum génération. Geochim. Cosmochim. Acta, 29, 1021-1049.
- Jean Burrus, 1997, contribution à l'étude du fonctionnement des systèmes pétroliers : apport d'une modélisation bi-dimensionnelle, rapport de thèse de doctorat
- Behar, F., Leblond, C. and Saint-Paul, C., 1989, Analyse quantitative des effluents de pyrolyseen milieu ouvert et fermé. Oil and Gas Science and Technology 44, 387-411.
- Burnham, A.K., Braun, R.L., Gregg, H.R., Samoun, A.M., 1987. Comparison of methods for measuring kerogen pyrolysis rates and fitting kinetic parameters. Energy and Fuels 1, 452.
- Burnham, A.K., Braun, R.L., Samoun, A.M., 1988. Further comparison of methods for measuring kerogen pyrolysis rates and fitting kinetic parameters. Organic Geochemistry 13 (4-6), 839-845.
- Lewan, M.D., 1997, Experiments on the role of water in petroleum formation. Geochimica et Cosmochimica Acta, 61, 3691-3723.
- Lewan, M.D. and Ruble, T.E., 2002, Comparison of petroleum génération kinetics by isothermal hydrous and non-isothermal open-system pyrolysis. Organic Geochemistry, 33, 1457-1475.
- Vandenbroucke, M., Behar, F. and Rudkiewicz, J.L., 1999, Kinetic modelling of petroleum formation and cracking: Implications from the high pressure/ high temperature elgin field (u.k., northsea). Organic Geochemistry, 30, 9, 1105-1125.
- Espitalié, J., Laporte, J.L., Madec, M., Marquis, F., Leplat, P., Paulet, J. and Boutefeu, A., 1977, Rapid method for source rock characterization, and for détermination of their petroleum potential and degree of évolution: Revue de l'Institut Francais du Petrole et Annales des Combustibles Liquides, 32/1, 23-42.
- Espitalié, J., Makadi, K. S. and Trichet, J., 1984, Role of the minerai matrix during kerogen pyrolysis. Org. Geochem. 6, 365-382.

On connaît des simulateurs de bassin (par exemple WO 2014/040622 A1) permettant notamment de déterminer la production d'hydrocarbures d'origine thermogénique, c'est-à-dire des hydrocarbures obtenus par transformation chimique de la matière organique sous l'effet des fortes températures qui règnent dans le sous-sol profond.

De manière générale, l'avancement d'une réaction chimique, telle que la transformation du kérogène, est déterminée par une loi de vitesse de réaction de la forme :

$$\frac{dx}{dt} = -kx^n \qquad (1)$$

avec x représentant la quantité de l'espèce chimique considérée, n l'ordre de la réaction, k la constante de vitesse de la réaction et t le temps.

De manière classique, la constante k de loi de vitesse de réaction est déterminée par une loi empirique. On connaît notamment la loi d'Arrhenius, exprimée sous la forme :

$$k = A.\exp(- E/RT) \qquad (2)$$

où A est le facteur de fréquence ou facteur pré-exponentiel, E l'énergie d'activation, R la constante des gaz parfaits et T la température. Les paramètres A et E sont appelés de manière classique « paramètres cinétiques » de la loi de vitesse de réaction.

Afin de simuler de manière fiable l'évolution de la transformation de la matière organique et donc la quantité d'hydrocarbures produite au cours du temps, il est important d'avoir une estimation réaliste des paramètres cinétiques (comme par exemple les paramètres cinétiques A et E de la loi d'Arrhénius) de l'équation de vitesse de réaction décrite ci-dessus.

De manière classique, on détermine les paramètres cinétiques d'une loi de vitesse de réaction au moyen d'expériences en laboratoire de maturation artificielle de la roche mère immature. La mise en œuvre de ces expériences nécessite de réaliser des prélèvements d'échantillons de la roche-mère dans un endroit du bassin étudié où celle-ci n'a pas encore commencé son processus de transformation thermique. Les expériences en laboratoire peuvent consister en des séquences de chauffe d'échantillons de matière organique en atmosphère inerte, telles que décrites dans les brevets EP 0691540 B1 (US 5843787) et FR 3021749 (US 2015/0346179), ou encore en une chauffe de la matière organique dans des tubes en or (cf. par exemple (Ungerer 1990)). Les temps expérimentaux plus réduits que dans le milieu naturel sont compensés par l'utilisation de températures plus élevées (entre 300 et 700°C pour le laboratoire contre 80 à 200°C dans le milieu naturel). A partir des mesures réalisées pendant ces expériences, on peut estimer les paramètres cinétiques associés à l'équation de vitesse de réaction décrite ci-dessus. Une fois cette détermination effectuée, la loi de vitesse de réaction telle que décrite ci-dessus peut être utilisée dans un modèle de bassin, qui simule l'évolution de la température au cours des temps géologiques, et estime ainsi l'avancement de la transformation de la roche-mère (et donc les volumes de pétrole générés) tout au long de ceux-ci. Il est toutefois connu (cf. notamment le document (Burrus, 1997)) qu'il n'existe pas une solution unique des paramètres cinétiques (tels que les paramètres A et E) permettant de reproduire les mesures réalisées au laboratoire. Autrement dit, plusieurs valeurs des paramètres cinétiques peuvent satisfaire les mesures de laboratoire, mais conduire à des prédictions de maturité aberrantes après extrapolation aux conditions géologiques réelles.

Par ailleurs, les expériences décrites ci-dessus sont coûteuses en termes de temps d'acquisition des données. En effet, chaque roche mère a sa propre cinétique et il faudrait ainsi répéter le processus d'acquisition de données pour chacune des roches-mères étudiées. De plus, la mise en œuvre de ces expériences nécessite des prélèvements de roche mère non mâture, ce qui n'est pas toujours possible en pratique. Ainsi, il est classique de recourir à des analogues de la roche mère étudiée, pour lesquels soit des mesures ont été déjà été réalisées ou même pour lesquels des paramètres cinétiques ont préalablement été déterminés (comme par exemple dans le document (Pepper and Corvi, 1995)). Néanmoins, il est possible que la réactivité de la roche-mère analogue ne soit pas parfaitement représentative de la réactivité de la roche-mère réellement présente dans le bassin étudié. Il se peut alors que les résultats de la simulation de bassin intégrant une loi de vitesse de réaction établie à partir d'analogues géologiques ne soient pas cohérents avec les marqueurs d'avancement de la réaction effectivement mesurés dans le bassin sédimentaire (tels que le taux de transformation de la matière organique, ou encore le Tmax qui correspond à la température du pic de libération des composés hydrocarbonés). Or cette incohérence conduit à une mauvaise estimation des volumes d'hydrocarbures générés par la roche-mère par la simulation de bassin. Il est alors nécessaire de mettre en cohérence à la fois la cinétique et l'histoire thermique du bassin.

A ce jour, il n'existe pas de méthode permettant une telle mise en cohérence, si bien que l'utilisateur de la simulation de bassin, lorsqu'il constate une incohérence doit tester, par une technique d'essais-erreurs, différentes hypothèses concernant les valeurs des paramètres cinétiques, jusqu'à obtenir une cohérence plus satisfaisante. Il s'ensuit des temps de calcul allongés (une simulation de bassin est requise pour chaque essai), ainsi qu'un résultat subjectif (l'utilisateur définit de manière plus ou moins arbitraire les valeurs des paramètres à tester, et qui plus est, les combinaisons de valeurs de paramètres cinétiques à tester).

La présente invention vise à pallier ces inconvénients. En effet, la présente invention permet de mettre jour, de manière automatique, sans arbitraire de la part de l'utilisateur, les paramètres cinétiques de la loi de vitesse de réaction définie ci-dessus, soit par des mesures de laboratoire, soit par référence à un analogue géologique dont la loi de vitesse de réaction a déjà été paramétrée, de sorte à le rendre cohérent avec l'histoire thermique du bassin et avec les mesures relatives à l'évolution de la maturation de la roche mère au cours des temps géologiques. La présente invention permet ainsi d'améliorer les prédictions de la simulation de bassin à l'échelle du bassin.

De manière générale, la présente invention vise à mettre à jour les paramètres cinétiques d'une loi de vitesse de réaction de manière à reproduire, de manière conjointe, des mesures réalisées à l'échelle de laboratoire (simulées ou directement mesurées) et des mesures réalisées à l'échelle du bassin, combinées à l'historique thermique issu de la simulation de bassin (calibré ou non calibré). Ainsi, le procédé selon l'invention permet de déterminer des paramètres cinétiques tenant compte de l'ensemble de l'information disponible sur le bassin étudié, contribuant ainsi à une meilleure prédiction du potentiel pétrolier du bassin étudié.

**Le procédé selon l'invention**

La présente invention concerne un procédé mis en œuvre par ordinateur pour déterminer la quantité et/ou la qualité des hydrocarbures présents dans un bassin sédimentaire, lesdits hydrocarbures ayant été générés par maturation de la matière organique d'une roche-mère dudit bassin, ledit bassin sédimentaire ayant subi une pluralité d'événements géologiques définissant une séquence d'états dudit bassin, au moyen d'une simulation numérique de bassin exécutée sur ordinateur, à partir de valeurs représentatives de la maturation artificielle d'un échantillon immature représentatif de ladite roche-mère et d'une séquence de températures de maturation artificielle. Le procédé comprend au moins les étapes suivantes :

A. on réalise des mesures de grandeurs physiques relatives audit bassin au moyen de capteurs, lesdites mesures comprenant au moins des mesures de propriétés pétrophysiques dudit bassin, des mesures représentatives de la thermicité dudit bassin pour lesdits états, des mesures d'avancement de la maturation de ladite matière organique dudit bassin ;

B. au moyen de ladite simulation numérique de bassin, on détermine un modèle de bassin pour chacun desdits états en fonction au moins desdites mesures desdites propriétés pétrophysiques, et on détermine au moins un historique des températures dudit bassin pour lesdits états ;

C. on détermine des valeurs des paramètres cinétiques d'une loi de vitesse de réaction représentative de l'évolution de la maturation de ladite matière organique pour chacun desdits états en minimisant à la fois :

- un écart entre des valeurs prédites par ladite loi de vitesse de réaction appliquée avec ledit historique de températures dudit bassin déterminé pour lesdits états, et lesdites mesures d'avancement de la maturation de ladite matière organique, et
- un écart entre des valeurs prédites par ladite loi de vitesse de réaction appliquée selon ladite séquence de températures pour ladite maturation artificielle, et lesdites valeurs représentatives de ladite maturation artificielle ;

D. on détermine ladite quantité et/ou ladite qualité desdits hydrocarbures à partir desdits paramètres cinétiques de ladite loi de vitesse de réaction mis à jour et dudit historique de températures pour lesdits états.

Avantageusement, à l'issue de l'étape B, on peut déterminer un historique des températures dudit bassin calibré en mettant à jour ledit historique des températures dudit bassin en fonction desdites mesures de ladite thermicité dudit bassin pour lesdits états, et on peut appliquer l'étape C au moyen dudit historique des températures calibré.

Selon une mise en œuvre de l'invention, ledit échantillon immature représentatif de ladite matière organique peut être directement prélevé dans ledit bassin.

Alternativement, ledit échantillon immature représentatif de ladite matière organique peut être prélevé dans un analogue géologique dudit bassin.

Selon une mise en œuvre de l'invention, lesdites valeurs représentatives de ladite maturation artificielle dudit échantillon immature peuvent correspondre à des mesures de maturation artificielle réalisées sur ledit échantillon immature.

Avantageusement, lesdites mesures de maturation artificielle peuvent être réalisées en plaçant ledit échantillon immature sous atmosphère inerte, et en le soumettant à ladite séquence de températures pour ladite maturation artificielle, puis en mesurant une quantité d'hydrocarbures libérés au cours de ladite séquence de températures pour ladite maturation artificielle.

Alternativement, lesdites valeurs représentatives de ladite maturation artificielle peuvent être obtenues à partir de ladite loi de vitesse de réaction appliquée à une première estimation desdits paramètres cinétiques.

Selon une mise en œuvre de l'invention, ladite minimisation est réalisée au moyen d'une méthode par gradients.

Selon une variante de l'invention, à partir de ladite quantité et/ou qualité desdits hydrocarbures dudit bassin, on peut définir un schéma d'exploitation dudit bassin, et on peut exploiter ledit bassin sédimentaire en fonction de ladite quantification et/ou dudit schéma.

En outre, l'invention concerne un produit programme d'ordinateur téléchargeable depuis un réseau de communication et/ou enregistré sur un support lisible par ordinateur et/ou exécutable par un processeur, comprenant des instructions de code de programme pour la mise en œuvre du procédé tel que décrit ci-dessus, lorsque ledit programme est exécuté sur un ordinateur.

D'autres caractéristiques et avantages du procédé selon l'invention, apparaîtront à la lecture de la description ci-après d'exemples non limitatifs de réalisations, en se référant aux Figures annexées et décrites ci-après.

**Présentation succincte des figures**

- la Figure 1 présente une carte représentative du taux de transformation T (%) obtenue au moyen d'un procédé selon l'art antérieur appliqué au Bassin de Paris.

- la Figure 2 présente une carte représentative du taux de transformation T (%) obtenue au moyen du procédé selon l'invention appliqué au Bassin de Paris.

- la Figure 3 présente une carte représentative du taux de transformation T (%) obtenue par extrapolation de valeurs mesurées aux puits forés dans le Bassin de Paris, extraite de (Espitalié, 1987).

**Description détaillée du procédé**

De façon générale, l'un des objets de l'invention concerne un procédé pour déterminer la quantité et/ou la qualité des hydrocarbures présents dans un bassin sédimentaire, les hydrocarbures ayant été générés par maturation de la matière organique d'une roche-mère du bassin, le bassin sédimentaire ayant subi une pluralité d'événements géologiques définissant une séquence d'états du bassin.

Le procédé selon l'invention est mis en œuvre au moyen d'une simulation numérique de bassin exécutée sur ordinateur. De manière classique, un simulateur de bassin permet de reconstituer des processus géologiques et/ou géochimiques ayant affecté le bassin depuis un temps géologique t jusqu'au temps actuel. De manière classique, la période sur laquelle on reconstitue l'histoire de ce bassin est discrétisée en événements géologiques, appelés états par la suite et notés $\{A_i\}_{i \in [\![0;n]\!]}$. Ainsi, deux états sont séparés par un événement géologique (correspondant par exemple à un dépôt sédimentaire particulier et pouvant s'étaler entre une centaine d'années et quelques millions d'années). Un simulateur de bassin repose sur une représentation maillée du bassin, aussi appelée « modèle de bassin », et le simulateur de bassin permet de déterminer un tel modèle pour chaque état. Ainsi, un simulateur de bassin permet de calculer des grandeurs physiques relatives au bassin étudié en chaque maille de la représentation maillée associée à chaque état. Parmi les grandeurs physiques estimées par un simulateur de bassin, on trouve la température, la pression, la porosité et la masse volumique de la roche contenue dans la maille considérée, les vitesses d'eau, et le TOC (ou concentration de la roche en matière organique). De manière classique, le simulateur de bassin utilisé permet, en outre, de calculer la quantité d'hydrocarbures d'origine thermogénique. Le calcul des vitesses d'eau peut être réalisé comme décrit dans le document (Marsily, 1986), et le calcul des autres quantités physiques citées ci-dessus peut être réalisé comme décrit dans le document (Schneider et al., 2000). Ainsi, la simulation de bassin consiste en la résolution d'un système d'équations différentielles décrivant l'évolution au cours du temps des grandeurs physiques étudiées. Pour ce faire, on peut par exemple utiliser une discrétisation par la méthode des volumes finis, comme décrit par exemple dans (Scheichl et al., 2003). Pour chaque état $A_i$, il est nécessaire de résoudre les équations par petits incréments de temps (i.e. avec un petit pas de temps $dt$) jusqu'à l'état suivant $A_{i+1}$. Conformément au principe des méthodes de volumes finis centrés sur les mailles, les inconnues sont discrétisées par une valeur constante par maille et les équations de conservation (masse ou chaleur) sont intégrées en espace sur chaque maille et en temps entre deux pas de temps successifs.

Les équations discrètes expriment alors que la quantité conservée dans une maille à un pas de temps donné est égale à la quantité contenue dans la maille au pas de temps précédent, augmentée des flux de quantités entrés dans la maille et réduite des flux de quantités sorties de la maille par ses faces, plus les apports extérieurs.

Le simulateur de bassin selon l'invention permet au moins de simuler l'évolution des températures au sein de chaque maille de la représentation maillée de chacun des modèles de bassin. Un exemple d'un tel simulateur de bassin est le logiciel TemisFlow™ (IFP Énergies nouvelles, France).

Le procédé selon l'invention comporte au moins les étapes décrites ci-après.

## 1. Mesures de grandeurs physiques

### 1.1. Mesures relatives à la thermicité du bassin

Au cours de cette étape, il s'agit d'acquérir des mesures relatives à l'histoire thermique du bassin, au moyen de capteurs. Ces mesures peuvent être réalisées dans au moins un puits traversant le bassin, pour lequel on acquiert des données de thermicité au cours du forage du puits et/ou de l'exploration et/ou de l'exploitation du bassin. Ces mesures peuvent aussi être réalisées à partir de prélèvements d'échantillons, prélevés par exemple dans un puits traversant le bassin. Ces données thermiques peuvent être des mesures de températures directes dans le bassin, des mesures de la réflectance de la vitrinite (qui traduit la maturité du bassin) et/ou des mesures de transformation de la matière organique du bassin sédimentaire.

Ainsi, les mesures de la thermicité actuelle peuvent être acquises pendant le forage et résulter de différentes méthodes, comme par exemple une mesure directe de la température tout au long du puits, l'estimation du gradient thermique par l'utilisation de sondes sur les couches superficielles des fonds océaniques, ou même encore la température de fond de puits donnée par « Drill Stem Test » (DST) mesurée dans le fluide de forage après l'arrêt des opérations. Toutes ces mesures renseignent sur le champ de température actuel dans le bassin sédimentaire.

On peut également utiliser des indicateurs géochimiques pouvant fournir des éléments quant aux paleotempératures qu'ont pu subir les formations géologiques. Parmi ceux-ci, la réflectance de la vitrinite est le plus largement répandu. Celle-ci est estimée grâce à la mesure de la réflexion de la lumière d'une lame mince en lumière polarisée. Les lames minces peuvent être préparées à partir de carottes prélevées dans le puits ou à partir de « cuttings » (résidus et fragments de roche qui remontent avec les boues pendant le forage). Dans la pratique, la mesure de la vitrinite est fonction du maximum de température qu'a subi l'échantillon.

On connaît aussi d'autres indicateurs géochimiques tels que l'index d'altération des Conodonts, les transformations minérales des argiles, la fission de l'apatite, les biomarqueurs ou encore les inclusions fluides. Ces marqueurs peuvent apporter des informations complémentaires à la vitrinite. On connaît notamment les inclusions fluides qui peuvent renseigner sur l'âge du maximum de température, ou encore la fission de l'apatite qui peut dater de manière absolue certains évènements géologiques.

### 1.2. Mesures relatives à la maturation de la roche mère du bassin

Au cours de cette étape, il s'agit d'acquérir des mesures relatives à la maturation de la roche-mère du bassin, au moyen de capteurs.

Ces mesures sont réalisées à partir d'échantillons de roche prélevées dans des puits (par carottages ou issus de déblais) traversant le bassin étudié, comme des puits d'exploration. Ces expériences peuvent être effectuées sur des échantillons de différente nature (roche brute, kérogène, extrait...). Il existe de nombreuses approches expérimentales permettant de d'étudier l'avancement et la nature des réactions de transformation de la matière organique.

Parmi les approches couramment utilisées, on peut citer des séquences de chauffe d'échantillons en atmosphère inerte, telles que réalisées au moyen du dispositif ROCK-EVAL™ (IFP Energies nouvelles) et décrites dans les brevets EP 0691540 B1 (US 5843787) et FR 3021749 (US 2015/0346179), ou encore des séquences de chauffe dans des tubes en or (Ungerer 1990), des expériences en milieu fermé anhydre (ex: pyrolyse en tube en or) ou en milieu fermé aqueux (Lewan, 1997; Lewan et Ruble, 2002). On mesure alors la quantité de composés hydrocarbonés libérés pendant cette séquence de chauffe, qui est donc une quantité fonction du temps et/ou de la température.

Les séquences de chauffe auxquelles est soumis un échantillon peuvent comprendre des rampes (avec des gradients de 0.1°C/minute à plusieurs dizaines de degrés par minute) et/ou bien des paliers isothermes (à 100°C, 250°C, 600°C, etc), ces paliers pouvant entre quelques heures à quelques semaines. Les rampes de chauffe se rapprochent le plus des conditions réelles, pour lesquelles on peut approximer l'augmentation de température du milieu géologique en fonction du temps par une constante. A partir d'une telle séquence de chauffe, selon une mise en œuvre de l'invention, on peut au moins déterminer la température pour laquelle la libération de composés hydrocarbonés est la plus forte (température du pic de libération des composés hydrocarbonés), classiquement notée Tmax, et qui est classiquement utilisée comme indicateur de maturité (Espitalié et al., 1977).

Selon une mise en œuvre de l'invention dans laquelle on utilise une séquence de chauffe avec température constante, on peut déterminer de manière analytique l'avancement de la transformation de la source en fonction du temps (en utilisant un formalisme du type de celui de la loi d'Arrhenius décrite ci-dessus par exemple).

**1.3 Mesures de grandeurs physiques relatives à la simulation de bassin**

Il s'agit de mesures qui sont requises pour l'exécution d'une simulation numérique de bassin, notamment pour remplir les mailles de la représentation maillée reproduisant le bassin sédimentaire étudié comme cela est décrit dans l'étape 2 ci-après. Ces mesures peuvent consister en des études d'affleurement, des campagnes d'acquisition sismique, des mesures dans des puits (par diagraphies par exemple), des analyses pétrophysique/géochimique de carottes prélevées in situ. A partir de ces mesures, on peut en déduire des propriétés pétrophysiques associées au bassin étudié, tels que le faciès, la porosité, la perméabilité, la saturation, ou encore la teneur en matière organique en des points de mesure du bassin.

**2. Simulation de bassin**

On détermine un modèle de bassin pour chaque d'état $\{A_i\}$ du bassin en fonction des mesures des grandeurs physiques décrites à l'étape 1.3, au moyen d'une simulation numérique de bassin.

Selon l'invention, on utilise pour ce faire un simulateur selon l'art antérieur. Une simulation de bassin comporte classiquement trois étapes :

- une étape de géo-modélisation qui consiste à construire une représentation maillée représentative du bassin étudié au temps actuel. Cette représentation maillée est le plus souvent structurée en couches, c'est-à-dire qu'un groupe de mailles est affecté à chaque couche géologique du bassin modélisé. Puis, chaque maille de cette représentation maillée est remplie par une ou plusieurs propriétés pétrophysiques, telles que la porosité, le faciès (argile, sable, etc) ou encore la teneur en matière organique. La construction de ce modèle se base sur des mesures de grandeurs physiques telles que décrites ci-dessus (cf. étape 1.3). Il s'agit au cours de cette étape de construire une représentation maillée du bassin étudié au temps actuel. Cette maquette du bassin est généralement représentée sur ordinateur, sous la forme d'un maillage ou grille, chaque maille étant caractérisée par une ou plusieurs propriétés relatives au bassin (telles que le faciès, la porosité, la perméabilité, la saturation, ou encore la teneur en matière organique au moment de la sédimentation).

- une étape de reconstruction structurale de l'architecture du bassin : il s'agit de reconstruire les architectures passées du bassin pour les différents états. Pour ce faire, on déforme la représentation maillée construite à l'étape précédente et qui représente le bassin au temps actuel afin de représenter l'évolution anti-chronologique de l'architecture du sous-sol au cours des temps géologiques, et ce pour les différents états $A_i$. On obtient ainsi une représentation maillée pour chaque état $A_i$.
  Selon un mode de réalisation de la présente invention, la reconstruction structurale peut être particulièrement simple si elle se base sur l'hypothèse que sa déformation résulte uniquement d'une combinaison de mouvements verticaux par compaction du sédiment ou par surrection ou affaissement de son socle. Cette technique, connue sous le terme de "backstripping" (ou « décompaction progressive du bassin » en français) est décrite par exemple dans (Steckler et Watts, 1978).
  Selon un autre mode de réalisation de la présente invention, dans le cas de bassins dont l'histoire tectonique est complexe, notamment dans le cas de bassins présentant des failles, il convient d'utiliser des techniques aux hypothèses moins restrictives, telles que la restauration structurale. Une telle restauration structurale est décrite par exemple dans le document FR 2 930 350 A (US 2009/0265152 A). La restauration structurale consiste à calculer les déformations successives que le bassin a subies, en intégrant les déformations dues à la compaction et celles qui résultent des forces tectoniques.

- une étape de simulation de bassin : il s'agit d'une simulation numérique d'une sélection de phénomènes physiques et chimiques se déroulant au cours de l'évolution du bassin et contribuant à la formation des pièges pétroliers, tels que décrit ci-dessus. Cette simulation de bassin s'effectue pour chaque état $A_i$, et s'appuie, pour chaque état $A_i$, sur la représentation maillée construite pour l'état $A_i$ considéré tel que décrit à l'étape précédente. Le simulateur de bassin détermine ainsi un modèle de bassin pour chaque état, chacune des mailles de ce modèle comprenant des grandeurs physiques, dont au moins la température pour la mise en œuvre du procédé selon l'invention, et de manière avantageuse, la pression, la porosité, la masse volumique de la roche, les vitesses d'eau, et le TOC (ou concentration de la roche en matière organique) etc. Un exemple d'un tel simulateur est le logiciel TemisFlow™ (IFP Énergies nouvelles, France).

A l'issue d'une telle simulation de bassin, on obtient un modèle de bassin pour chacun des états Ai, chaque maille de chacun de ces modèles étant remplie avec au moins une valeur de la température prédite par la simulation numérique de bassin.

On peut alors extraire un historique de la thermicité du bassin pour au moins lesdits états Ai. On peut par exemple extraire un historique des températures en des mailles particulières des modèles de bassin ainsi simulés (en général à la base du bassin sédimentaire), représentatif de l'évolution des températures en ces mailles au cours du temps, et au moins pour les états Ai simulés.

**3. Calibration thermique des modèles de bassin**

Cette étape est optionnelle. Au cours de cette étape, on calibre thermiquement les modèles de bassin, en ajustant l'historique thermique issu des modèles de bassin en fonction des mesures représentatives de la thermicité du bassin décrites à l'étape 1.1 ci-dessus (par exemple des mesures de température directe ou de réflectance de la vitrinite, mesurées dans des puits de forage ou sur des affleurements).

Selon une mise en œuvre de l'invention, on détermine un historique de la thermicité du bassin étudié, à partir des modèles de bassin remplis en température, déterminés pour chaque état à l'issue de l'étape précédente. On compare cet historique aux mesures relatives à l'histoire thermique du bassin préalablement réalisées in situ. Puis on met à jour l'historique de la thermicité issu de la simulation de bassin à partir de l'historique déterminé par les mesures de thermicité, en imposant les valeurs de températures mesurées aux points de mesure, et en interpolant ces valeurs en trois dimensions, et ce, en tenant compte du modèle thermique issu de la simulation de bassin. On pourra se référer au document FR 2 996 038 qui décrit notamment une telle méthode de calibration thermique.

A l'issue de cette étape, on obtient un historique des températures calibré du bassin étudié. Cet historique comprend au moins une valeur de température calibrée pour chacun desdits états Ai.

**4. Détermination des paramètres cinétiques d'une loi de vitesse de réaction de la maturation de la roche mère**

Selon l'invention et comme de manière classique, la loi de vitesse de réaction représentative de l'évolution de la maturation de la matière organique du bassin étudié est donnée par une formule du type :

$$\frac{dx}{dt} = -kx^n \qquad (1)$$

avec x représentant la quantité de l'espèce chimique considérée, n l'ordre de la réaction, k la constante de la loi de vitesse de réaction et t le temps.

Selon une mise en œuvre de l'invention, on utilise la loi d'Arrhenius pour représenter la constante de la loi de vitesse de réaction, soit :

$$k = A.\exp(-E/RT) \qquad (2)$$

où A est le facteur de fréquence ou facteur pré-exponentiel, E l'énergie d'activation, R la constante des gaz parfaits et T la température. De manière classique, les paramètres A et E de la loi d'Arrhenius sont appelés paramètres cinétiques de la loi de vitesse de réaction.

Selon une autre mise en œuvre de l'invention, on représente la constante de la loi de vitesse de réaction par une loi du type (Stainforth, 2009) :

$$k = \left(\frac{k_B T}{h}\right).\exp\left(-\frac{\Delta G}{RT}\right) \qquad (3)$$

pour laquelle, contrairement à la loi d'Arrhenius, le facteur pré-exponentiel $\left(A = \frac{k_B T}{h}\right)$ est variable et dépend de T, et E a une autre définition physique, et où $k_B$ est la constante de Boltzmann, h la constante de Planck et $\Delta G$ l'enthalpie libre molaire.

Selon l'invention, au cours de cette étape, on détermine les paramètres cinétiques de cette loi de vitesse de réaction en minimisant à la fois :

i. l'écart entre les valeurs prédites par la loi de vitesse de réaction, la loi étant appliquée avec l'historique de températures du bassin déterminé à l'étape 2 (historique de températures non calibré) ou 3 (historique de températures calibré), et les mesures d'avancement de la maturation de la matière organique réalisées in situ (cf étape 1.2 ci-dessus), et

ii. l'écart entre des valeurs prédites par la loi de vitesse de réaction choisie, appliquée selon une séquence de températures pour la maturation artificielle, et des valeurs représentatives de la maturation artificielle d'un échantillon immature représentatif de la roche-mère du bassin étudié obtenues pour la même séquence de températures de maturation artificielle.

La provenance des valeurs représentatives de la maturation artificielle d'un échantillon immature représentatif de la roche-mère du bassin étudié sont détaillées ci-après.

Selon une première variante de mise en œuvre de l'invention, les valeurs représentatives de la maturation artificielle d'un échantillon immature représentatif de la roche-mère du bassin étudié, résultent d'expériences de laboratoire réalisées sur un échantillon immature de la roche-mère du bassin directement prélevé dans le bassin étudié. Les expériences en laboratoire peuvent consister en des séquences de chauffe d'échantillons de matière organique en atmosphère inerte, telles que décrites dans les brevets EP 0691540 B1 (US 5843787) et FR 3021749 (US 2015/0346179), ou encore en une séquence de chauffe de la matière organique dans des tubes en or (Ungerer 1990).

Bien souvent toutefois, un tel échantillon immature de la matière organique, prélevé directement dans le bassin étudié n'est pas accessible. Selon une deuxième variante de mise en œuvre de l'invention, si un échantillon issu d'un analogue géologique au bassin étudié est disponible, les valeurs représentatives de la maturation artificielle d'un échantillon immature représentatif de la roche-mère du bassin étudié résultent d'expériences de laboratoire réalisées sur un échantillon prélevé dans un analogue géologique du bassin étudié. Les expériences en laboratoire peuvent consister en des séquences de chauffe d'échantillons de matière organique en atmosphère inerte, telles que décrites dans les brevets EP 0691540 B1 (US 5843787) et FR 3021749 (US 2015/0346179), ou encore en une séquence de chauffe de la matière organique dans des tubes en or (Ungerer 1990).

Selon une troisième variante de mise en œuvre de l'invention, lorsqu'aucun échantillon immature représentatif de la roche-mère du bassin n'est disponible, on peut s'appuyer sur des mesures de maturation artificielle d'un échantillon immature représentatif de la roche-mère du bassin étudié référencées dans la littérature, réalisées sur un échantillon immature provenant du bassin étudié, ou bien sur un échantillon immature provenant d'un analogue géologique du bassin étudié.

Selon une quatrième variante de mise en œuvre de l'invention dans laquelle on ne dispose pas de mesures de maturation artificielle réalisées sur un échantillon immature représentatif de la roche-mère du bassin étudié (provenant du bassin étudié ou bien d'un analogue géologique), mais que l'on dispose de valeurs de paramètres cinétiques représentatifs de la roche-mère du bassin étudié, on peut créer, de manière synthétique, des valeurs représentatives de la maturation artificielle de la roche mère étudiée, en appliquant une loi de vitesse de réaction paramétrée avec les valeurs des paramètres cinétiques disponibles et une séquence de températures représentative de la maturation artificielle. Ces valeurs des paramètres cinétiques peuvent avoir été prédéterminées à la mise en œuvre de la présente invention, ou bien provenir d'études faisant référence dans le domaine. On peut trouver un exemple d'une telle étude référençant des valeurs des paramètres cinétiques A et E de la loi d'Arrhénius dans le document (Pepper and Corvi, 1995). Selon cette variante de mise en œuvre de l'invention, à partir des valeurs des paramètres cinétiques issus de la littérature et applicables au bassin étudié, on simule des valeurs reproduisant une maturation artificielle de la roche-mère qui serait réalisée en laboratoire selon une séquence de températures caractéristique des mesures de maturation artificielle de laboratoire. On peut par exemple simuler, au moyen d'une loi de vitesse de réaction, une expérience de laboratoire qui se serait déroulée selon une séquence de températures comprenant une chauffe de l'échantillon pendant 15 minutes à 300°C suivie d'une augmentation de 25°C par minutes jusqu'à 700°C. D'autres histoires de chauffe peuvent également être utilisées, comme par exemple une température constante pendant différents intervalles de temps comme cela peut être fait pour des expériences en tube en or.

Ainsi, cette étape vise à mettre à jour les paramètres cinétiques de la loi de vitesse de réaction (comme par exemple les paramètres (A, E) de la loi d'Arrhenius), de sorte à reproduire à la fois des mesures réalisées à l'échelle de laboratoire (simulées ou directement mesurées) et des mesures réalisées à l'échelle du bassin, combinées à l'historique thermique issu de la simulation de bassin (calibré ou non calibré).

De manière classique, la minimisation selon l'invention est réalisée en deux étapes : on détermine des valeurs initiales pour les paramètres cinétiques (cf. étape 4.1 ci-dessous), puis on met à jour les paramètres cinétiques (cf. étape 4.2 ci-dessous).

**4.1. Estimation des valeurs initiales des paramètres cinétiques**

Au cours de cette sous-étape, il s'agit de déterminer des valeurs initiales pour les paramètres cinétiques de la loi de vitesse de réaction. Cette détermination se fait en fonction des mesures de maturation artificielle disponibles (cf. ci-dessus).

Selon les première, deuxième et troisième variantes de mise en œuvre de l'invention décrites ci-dessus, pour lesquelles on dispose de mesures de la maturation artificielle réalisées sur un échantillon immature représentatif de la roche-mère du bassin étudié (directement prélevé ou provenant d'un analogue), on estime des valeurs initiales des paramètres cinétiques de la loi de vitesse de réaction choisie par inversion des mesures de maturation artificielle. Selon une mise en œuvre de l'invention selon laquelle la loi de vitesse de réaction choisie est la loi d'Arrhenius, on détermine, à partir de ces mesures de maturation artificielle, les paramètres A et E de cette loi. Selon une mise en œuvre de l'invention, la procédure d'inversion des mesures de maturation artificielle en laboratoire se déroule en deux phases :

- dans une première phase, le paramètre A est fixé et la distribution du paramètre E est optimisée par une méthode des moindres carrés ;

- dans une seconde phase, différentes valeurs du paramètre A sont testées afin d'obtenir une calibration optimale entre une courbe théorique et les mesures de maturation.

Selon la quatrième variante de mise en œuvre de l'invention décrite ci-dessus, pour laquelle on simule de manière synthétique des valeurs de mesures de maturation artificielle à partir de valeurs de paramètres cinétiques prédéterminées, on utilise directement les valeurs de ces paramètres pour initialiser la loi de vitesse réaction choisie. On peut trouver un exemple d'une telle étude référençant des valeurs des paramètres cinétiques A et E de la loi d'Arrhénius dans le document (Pepper and Corvi, 1995).

On obtient à l'issue de cette étape une loi de vitesse de réaction paramétrée, avec des valeurs de paramètres cinétiques initiales. Ces valeurs de paramètres vont être optimisées au cours de l'étape 4.2 décrite ci-dessous.

**4.2. Mise à jour des valeurs des paramètres cinétiques**

Au cours de cette étape, on met à jour les valeurs des paramètres cinétiques de la loi de vitesse de réaction.

Selon les première, deuxième et troisième variante de mise en œuvre de l'invention pour lesquelles on dispose de mesures reproduisant la maturation d'un échantillon immature directement prélevé dans le bassin étudié ou provenant d'un analogue géologique au bassin, la mise à jour selon l'invention est réalisée de manière à minimiser à la fois :

- l'écart entre les valeurs prédites par la loi de vitesse de réaction, la loi étant appliquée avec l'historique de températures du bassin déterminé à l'étape 2 (historique de températures non calibré) ou 3 (historique de températures calibré), et les mesures d'avancement de la maturation de la matière organique réalisées in situ (cf étape 1.2 ci-dessus), et

- un écart entre les valeurs prédites par la loi de vitesse de réaction, la loi étant appliquée avec la séquence de températures utilisée pour réaliser les expériences de maturation artificielle, et les valeurs elles-mêmes des mesures reproduisant ladite maturation artificielle.

Selon la quatrième variante de mise en œuvre de l'invention pour laquelle on ne dispose pas de mesures directes de la maturation d'un échantillon immature mais de mesures de maturation simulées numériquement, on applique la mise à jour des paramètres cinétiques de la manière suivante en minimisant à la fois :

- l'écart entre les valeurs prédites par la loi de vitesse de réaction, la loi étant appliquée avec l'historique de températures du bassin déterminé à l'étape 2 (historique de températures non calibré) ou 3 (historique de températures calibré), et les mesures d'avancement de la maturation de la matière organique réalisées in situ (cf étape 1.2 ci-dessus), et

- l'écart entre les valeurs prédites par la loi de vitesse de réaction, la loi étant appliquée avec la séquence de températures utilisée pour simuler une expérience de maturation artificielle, et les valeurs de cette maturation artificielle simulée. Ainsi, selon cette quatrième variante de mise en œuvre de l'invention, les valeurs simulées de maturation artificielle servent comme contraintes pour stabiliser l'inversion des mesures d'avancement.

Ainsi, au cours de cette étape, on met à jour les paramètres cinétiques de la loi de vitesse de réaction (comme par exemple les paramètres A et E de la loi d'Arrhenius), de sorte à reproduire à la fois des mesures à l'échelle de laboratoire (simulées ou directement mesurées) et des mesures réalisées à l'échelle du bassin, combinées à l'historique thermique issu de la simulation de bassin (calibré ou non calibré).

La méthode de mise à jour des paramètres cinétiques peut-être quelconque (méthode bayésienne, par gradient, par régression linéaire...).

Selon une mise en œuvre de l'invention, on utilise une méthode par gradients, telle qu'implémentée par exemple dans le logiciel CougarFlow ™ (IFP Energies nouvelles, France). Elle utilise une méthode de plus grande pente pour déterminer le minimum d'une fonction objectif. Ce type de méthode donne généralement accès à un unique optimum de la fonction coût qui peut être un minimum local. Elle ne vise donc qu'un objectif d'ajustement du modèle.

Selon une autre mise en œuvre de la méthode, on utilise une approche bayésienne. Cette méthode vise à estimer une distribution de probabilité a posteriori de la paramétrisation d'un modèle. Cette distribution donne ainsi accès à un modèle optimal mais également à des informations qui permettent d'estimer des incertitudes sur la paramétrisation du modèle et de tenir compte de ces dernières sur les prédictions réalisées. En outre, cette méthode permet d'accéder à un optimum global, mais peut en revanche nécessiter de réaliser un plus grand nombre de simulations. Les distributions de probabilité peuvent par la suite être utilisées pour déterminer une paramétrisation optimale ainsi que pour propager l'ensemble des incertitudes de paramétrisation du modèle sur les valeurs des propriétés à prédire.

## 5. Détermination de la quantité et/ou de la qualité des hydrocarbures

Au cours de cette étape, on détermine la quantité et/ou la qualité desdits hydrocarbures présents dans le bassin sédimentaire étudié, à partir des paramètres cinétiques de ladite loi de vitesse de réaction mis à jour et de l'historique de températures issu de l'étape 2 (historique de températures calibré)) ou de l'étape 3 (historique de températures non calibré).

Selon l'invention, on applique la loi de vitesse de réaction mise à jour dans chacune des mailles de chacun des modèles de bassin comprenant de la matière organique, de manière à calculer le niveau de transformation de cette matière organique au cours du temps (pour chaque état Ai), et en tenant compte de l'historique de températures issu de l'étape 2 (historique de températures non calibré) ou, préférentiellement, de l'étape 3 (historique de températures calibré).

On détermine ainsi la quantité et/ou la qualité des hydrocarbures présents dans le bassin à l'actuel, et ce de manière plus fiable qu'avec un simulateur de bassin selon l'art antérieur, car la loi de vitesse de réaction est paramétrée de manière à tenir compte de l'ensemble des informations cinétiques disponibles, dont des mesures à l'échelle de laboratoire (simulées ou directement mesurées) et des mesures réalisées à l'échelle du bassin. De plus, selon une mise en œuvre de l'invention, on peut prendre en compte un modèle thermique calibré (tel que décrit à l'étape 3 est possible), ce qui améliore encore la fiabilité des résultats obtenus en termes de quantité et de qualité des hydrocarbures présents dans le bassin.

Selon une mise en œuvre de l'invention, on utilise en sus des informations concernant l'épaisseur de la roche-mère, sa richesse en matière organique, les fluides générés afin que la prédiction de la quantité et/ou la qualité des hydrocarbures présents dans le bassin soit la plus fiable possible.

Ainsi, le procédé selon l'invention permet d'éviter au spécialiste de procéder par une technique d'essais-erreurs pour tester différentes hypothèses jusqu'à obtenir une cohérence avec les mesures réalisées in situ, tout en continuant de satisfaire les mesures de laboratoire. Il s'ensuit des temps de calcul raccourcis (il n'y a plus de simulation de bassin a lancé pour chaque essai), ainsi qu'un résultat plus objectif (l'utilisateur ne définit plus de manière plus ou moins arbitraire les valeurs des paramètres à tester, et qui plus est, les combinaisons de valeurs de paramètres à tester).

Ainsi, la présente invention permet de mettre jour, de manière automatique, sans arbitraire de la part de l'utilisateur, les paramètres cinétiques de la loi de vitesse de réaction définie ci-dessus de sorte à le rendre cohérent avec l'histoire thermique du bassin et avec les mesures relatives à l'évolution de la maturation de la roche mère au cours des temps géologiques. La présente invention permet ainsi d'améliorer les prédictions de la simulation de bassin à l'échelle du bassin.

## 6. Exploitation des hydrocarbures de la formation

A l'issue des précédentes étapes, on dispose au moins de la quantité et/ou la qualité des hydrocarbures présents dans chacune des mailles de la dite représentation maillée au temps actuel.

En outre, en fonction du simulateur de bassin utilisé pour mettre en œuvre l'invention, on peut disposer par exemple d'informations sur :

    i. la mise en place des couches sédimentaires,

    ii. leur compaction sous l'effet du poids des sédiments sus-jacents,

iii. leur réchauffement au cours de leur enfouissement,

iv. les modifications de pressions de fluides résultant de cet enfouissement,

v. la formation des hydrocarbures formés par thermogénèse,

vi. le déplacement de ces hydrocarbures dans le bassin sous l'effet de la flottabilité, de la capillarité, des différences de gradients de pression les écoulements souterrains,

A partir de telles informations, le spécialiste a alors connaissance des zones dudit bassin, correspondant à des mailles de ladite représentation maillée au temps actuel dudit bassin, comportant des hydrocarbures, ainsi que la teneur, la nature et la pression des hydrocarbures qui y sont piégés. Le spécialiste est alors en mesure de sélectionner les zones du bassin étudié présentant le meilleur potentiel pétrolier.

L'exploitation pétrolière du bassin peut alors prendre plusieurs formes, notamment :

- la réalisation de forages d'exploration dans les différentes zones sélectionnées comme présentant le meilleur potentiel, afin de confirmer ou infirmer le potentiel estimé préalablement, et d'acquérir de nouvelles données pour alimenter de nouvelles études plus précises,

- la réalisation de forages d'exploitation (puits producteurs ou injecteurs) pour la récupération des hydrocarbures présents au sein du bassin sédimentaire dans les zones sélectionnées comme présentant le meilleur potentiel.

**Equipement et produit programme d'ordinateur**

Le procédé selon l'invention est mis en œuvre au moyen d'un équipement (par exemple un poste de travail informatique) comprenant des moyens de traitement des données (un processeur) et des moyens de stockage de données (une mémoire, en particulier un disque dur), ainsi qu'une interface d'entrée et de sortie pour saisir des données et restituer les résultats du procédé.

Les moyens de traitement de données sont configurés pour réaliser les étapes suivantes :

- au moyen d'une simulation numérique de bassin, on détermine un modèle de bassin pour chacun des états Ai en fonction au moins des mesures des propriétés pétrophysiques, et on détermine au moins un historique des températures du bassin pour lesdits états ;
- on détermine les valeurs des paramètres cinétiques de la loi de vitesse de réaction représentative de l'évolution de la maturation de ladite matière organique pour chacun desdits états en minimisant à la fois :

    o un écart entre des valeurs prédites par la loi de vitesse de réaction appliquée avec l'historique de températures du bassin déterminé pour les états, et les mesures d'avancement de la maturation de la matière organique, et

    o un écart entre des valeurs prédites par ladite loi de vitesse de réaction appliquée selon la séquence de températures pour la maturation artificielle, et les valeurs représentatives de la maturation artificielle ;

- on détermine la quantité et/ou la qualité des hydrocarbures à partir des paramètres cinétiques de la loi de vitesse de réaction mis à jour et de l'historique de températures pour les états Ai.

En outre, l'invention concerne un produit programme d'ordinateur téléchargeable depuis un réseau de communication et/ou enregistré sur un support lisible par ordinateur et/ou exécutable par un processeur, comprenant des instructions de code de programme pour la mise en œuvre du procédé tel que décrit précédemment, lorsque ledit programme est exécuté sur un ordinateur.

**Exemple de réalisation**

Les caractéristiques et avantages du procédé selon l'invention apparaîtront plus clairement à la lecture de l'exemple d'application ci-après.

Le procédé selon l'invention est appliqué au bassin de Paris (Fance). Ce bassin est une cuvette sédimentaire de 140 000 km2, 500 km d'Est en Ouest sur 300 du nord au sud, formée de couches sédimentaires concentriques typique des bassins intracratoniques. Jusqu'à aujourd'hui, plus de 240 millions de barils ont été récupérés dans les 52 champs en exploitation.

L'exploration et l'exploitation de ce bassin ont donné lieu à de nombreux prélèvements in situ d'échantillons, notamment analysés au moyen du dispositif ROCK-EVAL® (IFP Energies nouvelles, France), tel que décrit dans le brevet EP 2342557 (US 8796035).

Aux fins de la mise en œuvre du procédé selon l'invention, la maquette numérique représentative du bassin de Paris couvre l'ensemble du bassin sédimentaire et l'ensemble des dépôts, de la surface au socle. La définition horizontale, c'est-à-dire la taille d'une cellule unitaire, est de 2km x2km. Chronologiquement, le modèle numérique reproduit une succession de 38 évènements géologiques, comprenant 31 dépôts de formations sédimentaires et 7 évènements érosifs.

Selon une mise en œuvre de l'invention, l'histoire thermique du modèle est calibrée sur un grand volume de données, à savoir plusieurs dizaines de puits contenant des informations de température mesurée et de vitrinite. A ces informations classiques viennent s'ajouter des contraintes moins courantes que sont les inclusions fluides et des « *clumped isotopes »,* qui sont des Paleo-Thermo-Chronomètres, c'est-à-dire que ces mesures renseignent sur la température observée dans une formation à un certain âge.

Les principales roches mères à l'origine des hydrocarbures en place, sont Toarcienne et Hettangienne. Le procédé selon l'invention a été appliqué à la roche-mère Hettangienne, mais aurait très bien pu être mis en œuvre pour la seconde roche mère. La roche mère étudiée est un roche mère marine, de type II, ayant une teneur en carbone organique modérée (de 0,7% à 1,2%).

Le procédé selon l'invention est appliqué selon la quatrième variante de mise en œuvre de l'invention, pour laquelle les valeurs représentatives de la maturation artificielle sont simulées numériquement à partir de valeurs de paramètres cinétiques pré-déterminées. En effet le bassin parisien étant bien connu, il est possible d'utiliser des valeurs de paramètres cinétiques connues dans la littérature pour créer des valeurs simulant des mesures de maturation artificielle.

La loi de vitesse de réaction choisie est la loi d'Arrhénius (cf. équation 2). Les mesures d'avancement disponibles correspondent à 11 valeurs de mesure du Tmax de la roche-mère Hettangienne, spatialement bien distribuées sur l'ensemble du bassin, au niveau des zones prospectives.

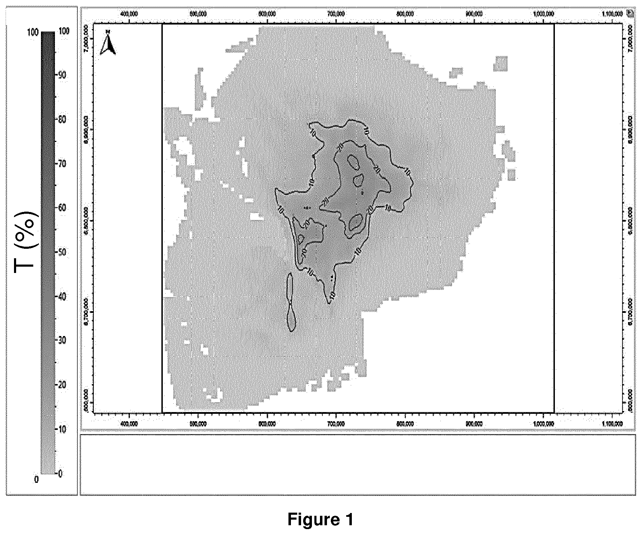

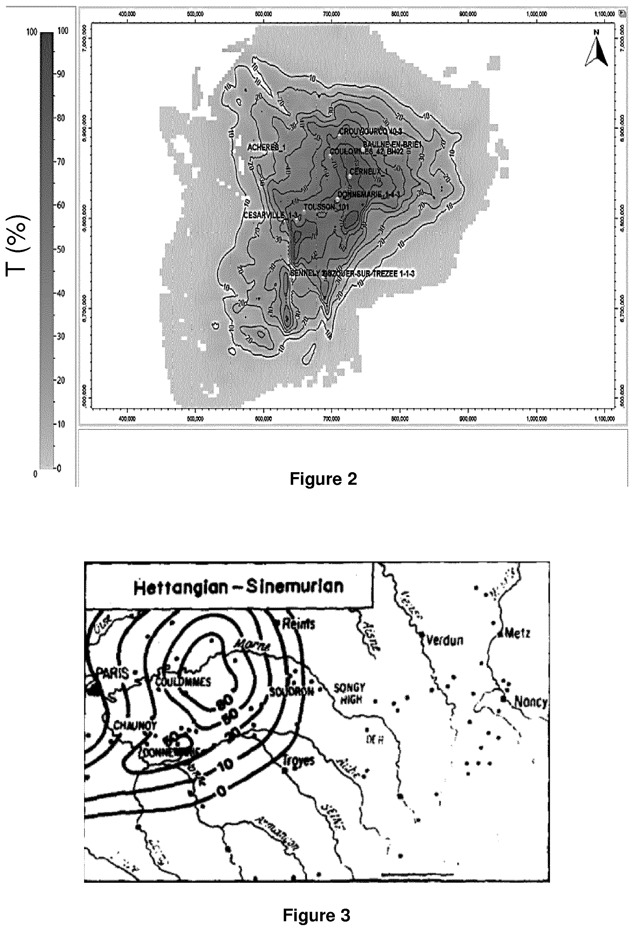

Le Tableau 1 compare les valeurs de TMax mesurées dans les échantillons de roche-mère et les valeurs de Tmax déterminées par un procédé selon l'art antérieur. On peut constater un certain écart entre les prédcitions de TMax selon l'art l'art antérieur et les Tmax mesurés. La Figure 1 présente une carte de taux de transformation obtenue au présent pour la roche mère Hettangienne par l'application d'un procédé selon l'art antérieur. On peut observer que le procédé selon l'art antérieur ne prédit des valeurs de taux de transformation d'une vingtaine de pourcent au maximum dans le centre du bassin. Ces valeurs ne sont pas en accord avec les mesures effectuées aux puits forés dans le Bassin de Paris. En effet, la carte présentée en Figure 3 (extraite de (Espitalié, 1987)), qui a été établie à partir d'extrapolation des mesures aux puits, montre plutôt un maximum autour de 80%.

| | Mesuré | Simulé |
|---|---|---|
| Achere | 427 | 416.75 |
| Baulnes | 439 | 419.75 |
| Cerneux | 441 | 426.75 |
| Cesarville | 433 | 418 |
| Charmottes | 438 | 419.75 |
| Crouy | 434 | 418.75 |
| Malnoue | 445 | 423.5 |
| Nantouillet | 438 | 417.5 |
| Ouzouer | 434 | 417.25 |
| Roches | 437 | 418.5 |
| Tousson | 436 | 418 |

Tableau 1

Le Tableau 2 compare les valeurs de TMax mesurées dans les échantillons de roche-mère et les valeurs de Tmax déterminées par le procédé selon l'invention, obtenues en minimisant à la fois les mesures à l'échelle du bassin et les valeurs de maturation artificielle (simulées à l'échelle du laboratoire). On peut observer un meilleur calage que selon l'art antérieur. La Figure 2 présente la carte de taux de transformation obtenue par application du procédé selon l'invention. Les ordres de grandeur sont comparables à ceux estimés par (Espitalié, 1987 ; cf. Figure 3). De plus, de par l'intégration de tous les phénomènes physico-chimiques engendrés par la géologie, la présente invention offre une spatialisation plus fine mais aussi une information sur l'ensemble du bassin.

|  | Mesuré | Simulé |
|---|---|---|
| Achere | 427 | 428.5 |
| Baulnes | 439 | 437.5 |
| Cerneux | 441 | 442.25 |
| Cesarville | 433 | 437 |
| Charmottes | 438 | 437.5 |
| Crouy | 434 | 437.25 |
| Malnoue | 445 | 439 |
| Nantouillet | 438 | 436 |
| Ouzouer | 434 | 434.25 |
| Roches | 437 | 437.25 |
| Tousson | 436 | 437 |

Tableau 2

Ainsi, la présente invention permet de mettre à jour, de manière automatique, sans arbitraire de la part de l'utilisateur, les paramètres cinétiques d'une loi de vitesse de réaction afin d'assurer la cohérence avec l'ensemble des mesures réalisées. En effet, la présente invention permet de tenir compte, en plus des mesures d'avancement relatives à l'histoire thermique du bassin, de valeurs représentatives de la maturation artificielle de la roche-mère étudiée (réalisées sur un échantillon prélevé dans le bassin étudié ou bien dans un analogue géologique). La présente invention permet ainsi d'améliorer les prédictions de la simulation de bassin à l'échelle du bassin.

## Revendications

1. Procédé mis en œuvre par ordinateur pour déterminer la quantité et/ou la qualité des hydrocarbures présents dans un bassin sédimentaire, lesdits hydrocarbures ayant été générés par maturation de la matière organique d'une roche-mère dudit bassin, ledit bassin sédimentaire ayant subi une pluralité d'événements géologiques définissant une séquence d'états dudit bassin, au moyen d'une simulation numérique de bassin exécutée sur ordinateur, à partir de valeurs représentatives de la maturation artificielle d'un échantillon immature représentatif de ladite roche-mère et d'une séquence de températures de maturation artificielle, le procédé comprenant au moins les étapes suivantes :

   A. on réalise des mesures de grandeurs physiques relatives audit bassin au moyen de capteurs, lesdites mesures comprenant au moins des mesures de propriétés pétrophysiques dudit bassin, des mesures représentatives de la thermicité dudit bassin pour lesdits états, des mesures d'avancement de la maturation de ladite matière organique dudit bassin ;
   B. au moyen de ladite simulation numérique de bassin, on détermine un modèle de bassin pour chacun desdits états en fonction au moins desdites mesures desdites propriétés pétrophysiques, et on détermine au moins un historique des températures dudit bassin pour lesdits états ;
   C. on détermine des valeurs des paramètres cinétiques d'une loi de vitesse de réaction représentative de l'évolution de la maturation de ladite matière organique pour chacun desdits états en minimisant à la fois :

      i. un écart entre des valeurs prédites par ladite loi de vitesse de réaction appliquée avec ledit historique de températures dudit bassin déterminé pour lesdits états, et lesdites mesures d'avancement de la maturation de ladite matière organique, et
      ii. un écart entre des valeurs prédites par ladite loi de vitesse de réaction appliquée selon ladite séquence de températures pour ladite maturation artificielle, et lesdites valeurs représentatives de ladite maturation artificielle ;

   D. on détermine ladite quantité et/ou ladite qualité desdits hydrocarbures à partir desdits paramètres cinétiques de ladite loi de vitesse de réaction mis à jour et dudit historique de températures pour lesdits états.

**2.** Procédé selon la revendication 1, dans lequel, à l'issue de l'étape B, on détermine un historique des températures dudit bassin calibré en mettant à jour ledit historique des températures dudit bassin en fonction desdites mesures de ladite thermicité dudit bassin pour lesdits états, et dans lequel on applique l'étape C au moyen dudit historique des températures calibré.

**3.** Procédé selon l'une des revendications précédentes, dans lequel ledit échantillon immature représentatif de ladite matière organique a été directement prélevé dans ledit bassin.

**4.** Procédé selon l'une des revendications 1 à 2, dans lequel ledit échantillon immature représentatif de ladite matière organique a été prélevé dans un analogue géologique dudit bassin.

**5.** Procédé selon l'une des revendications précédentes, dans lequel lesdites valeurs représentatives de ladite maturation artificielle dudit échantillon immature correspondent à des mesures de maturation artificielle réalisées sur ledit échantillon immature.

**6.** Procédé selon la revendication 5, dans lequel lesdites mesures de maturation artificielle sont réalisées en plaçant ledit échantillon immature sous atmosphère inerte, et en le soumettant à ladite séquence de températures pour ladite maturation artificielle, puis en mesurant une quantité d'hydrocarbures libérés au cours de ladite séquence de températures pour ladite maturation artificielle.

**7.** Procédé selon l'une des revendications 1 à 4, dans lequel lesdites valeurs représentatives de ladite maturation artificielle ont été obtenues à partir de ladite loi de vitesse de réaction appliquée à une première estimation desdits paramètres cinétiques.

**8.** Procédé selon l'une des revendications précédentes, dans lequel ladite minimisation est réalisée au moyen d'une méthode par gradients.

**9.** Procédé selon l'une des revendications précédentes, dans lequel, à partir de ladite quantité et/ou qualité desdits hydrocarbures dudit bassin, on définit un schéma d'exploitation dudit bassin, et on exploite ledit bassin sédimentaire en fonction de ladite quantification et/ou dudit schéma.

**10.** Produit programme d'ordinateur téléchargeable depuis un réseau de communication et/ou enregistré sur un support lisible par ordinateur et/ou exécutable par un processeur, comprenant des instructions de code de programme qui, lorsque le programme est exécuté par un ordinateur, conduisent celui-ci à mettre en œuvre le procédé selon l'une des revendications précédentes.

**Patentansprüche**

**1.** Computerimplementiertes Verfahren zur Bestimmung der Menge und/oder Qualität der Kohlenwasserstoffe, die in einem Sedimentbecken vorhanden sind, wobei die Kohlenwasserstoffe durch Reifung des organischen Materials eines Muttergesteins des Beckens erzeugt wurden, wobei das Sedimentbecken eine Mehrzahl von geologischen Ereignissen erfahren hat, die eine Abfolge von Zuständen des Beckens definieren, mittels einer digitalen Beckensimulation, die am Computer ausgeführt wird, ausgehend von Werten, die für die künstliche Reifung einer unreifen Probe repräsentativ sind, die für das Muttergestein repräsentativ ist, und einer Abfolge von Temperaturen künstlicher Reifung, wobei das Verfahren mindestens die folgenden Schritte umfasst:

A. Es werden Messungen physikalischer Größen bezogen auf das Becken mittels Sensoren durchgeführt, wobei die Messungen wenigstens Messungen petrophysikalischer Eigenschaften des Beckens, Messungen, die für die Thermizität des Beckens für die Zustände repräsentativ sind, Messungen des Voranschreitens der Reifung des organischen Materials des Beckens umfassen;

B. Mittels der digitalen Beckensimulation wird ein Beckenmodell für jeden der Zustände in Abhängigkeit von wenigstens den Messungen der petrophysikalischen Eigenschaften bestimmt, und es wird wenigstens ein Verlauf der Temperaturen des Beckens für die Zustände bestimmt;

C. Es werden Werte der kinetischen Parameter eines Reaktionsgeschwindigkeitsgesetzes, das für die Entwicklung der Reifung des organischen Materials repräsentativ ist, für jeden der Zustände bestimmt, wobei Folgendes zugleich minimiert wird:

i. eine Abweichung zwischen Werten, die durch das Reaktionsgeschwindigkeitsgesetz vorhergesagt werden, das mit dem Verlauf von Temperaturen des Beckens angewandt wird, der für die Zustände bestimmt wird, und den Messungen des Voranschreitens der Reifung des organischen Materials, und

ii. eine Abweichung zwischen Werten, die durch das Reaktionsgeschwindigkeitsgesetz vorhergesagt werden, das gemäß der Abfolge von Temperaturen für die künstliche Reifung angewandt wird, und den Werten, die für die künstliche Reifung repräsentativ sind;

D. Es wird die Menge und/oder Qualität der Kohlenwasserstoffe ausgehend von den aktualisierten kinetischen Parametern des Reaktionsgeschwindigkeitsgesetzes und dem Verlauf von Temperaturen für die Zustände bestimmt.

2. Verfahren nach Anspruch 1, wobei nach dem Schritt B ein kalibrierter Verlauf der Temperaturen des Beckens bestimmt wird, wobei der Verlauf der Temperaturen des Beckens in Abhängigkeit von den Messungen der Thermizität des Beckens für die Zustände aktualisiert wird, und wobei der Schritt C auf das Mittel des kalibrierten Verlaufs der Temperaturen angewandt wird.

3. Verfahren nach einem der vorhergehenden Ansprüche, wobei die unreife Probe, die für das organische Material repräsentativ ist, im Becken direkt entnommen wurde.

4. Verfahren nach einem der Ansprüche 1 bis 2, wobei die unreife Probe, die für das organische Material repräsentativ ist, in einem geologischen Analog des Beckens entnommen wurde.

5. Verfahren nach einem der vorhergehenden Ansprüche, wobei die Werte, die für die künstliche Reifung der unreifen Probe repräsentativ sind, Messungen künstlicher Reifung entsprechen, die an der unreifen Probe durchgeführt werden.

6. Verfahren nach Anspruch 5, wobei die Messungen künstlicher Reifung durchgeführt werden, indem die unreife Probe unter Inertatmosphäre gestellt und der Abfolge von Temperaturen für die künstliche Reifung unterzogen wird, dann indem eine Menge von Kohlenwasserstoffen gemessen wird, die im Verlauf der Abfolge von Temperaturen für die künstliche Reifung freigesetzt werden.

7. Verfahren nach einem der Ansprüche 1 bis 4, wobei die Werte, die für die künstliche Reifung repräsentativ sind, ausgehend von dem Reaktionsgeschwindigkeitsgesetz erhalten wurden, das auf eine erste Schätzung der kinetischen Parameter angewandt wurde.

8. Verfahren nach einem der vorhergehenden Ansprüche, wobei die Minimierung mittels eines Gradientenverfahrens durchgeführt wird.

9. Verfahren nach einem der vorhergehenden Ansprüche, wobei ausgehend von der Menge und/oder Qualität der Kohlenwasserstoffe des Beckens ein Schema zur Erschließung des Beckens festgelegt und das Sedimentbecken in Abhängigkeit von der Quantifizierung und/oder dem Schema erschlossen wird.

10. Computerprogrammprodukt, das aus einem Kommunikationsnetz heruntergeladen werden kann und/oder auf einem computerlesbaren Datenträger gespeichert ist und/oder von einem Prozessor ausgeführt werden kann, das Programmcode-Anweisungen umfasst, die bei Ausführung des Programms durch einen Computer diesen dazu veranlassen, das Verfahren nach einem der vorhergehenden Ansprüche durchzuführen.

**Claims**

1. Method executed by a computer for determining the quantity and/or the quality of the hydrocarbons present in a sedimentary basin, said hydrocarbons having been generated by maturing of the organic material of a mother rock of said basin, said sedimentary basin having undergone a plurality of geological events defining a sequence of states of said basin, by means of a numerical basin simulation executed on computer, on the basis of values representing the artificial maturing of an immature sample representing said mother rock and a sequence of artificial maturing temperatures, the method comprising at least the following steps:

A. physical parameters relating to said basin are measured by means of sensors, said measurements comprising at least measurements of petrophysical properties of said basin, measurements representing the thermicity of said basin for said states, measurements of the advance of the maturing of said organic material of said basin;

B. by means of said numerical basin simulation, a basin model is determined for each of said states as a function at least of said measurements of said petrophysical properties and at least one historical record of the temperatures of said basin for said states is determined;

C. values of kinetic parameters of a reaction rate law representing the evolution of the maturing of said organic material for each of said states are determined, minimizing simultaneously:

> i. a difference between values predicted by said reaction rate law applied with said historical record of temperatures of said basin determined for said states, and said measurements of the advance of the maturing of said organic material, and
>
> ii. a difference between values predicted by said reaction rate law applied according to said sequence of temperatures for said artificial maturing and said values representing said artificial maturing;

> D. said quantity and/or said quality of said hydrocarbons is/are determined from said kinetic parameters of said updated reaction rate law and said historical record of temperatures for said states.

2. Method according to Claim 1 in which at the end of step B a historical record of the temperatures of said basin is determined and calibrated by updating said historical record of the temperatures of said basin as a function of said measurements of said thermicity of said basin for said states and in which the step C is applied by means of said calibrated historical record of the temperatures.

3. Method according to either one of the preceding claims in which said immature sample representing said organic material has been directly sampled in said basin.

4. Method according to either one of Claims 1 and 2 in which said immature sample representing said organic material has been sampled in a geological analogue of said basin.

5. Method according to any one of the preceding claims in which said values representing said artificial maturing of said immature sample correspond to measurements of artificial maturing made on said immature sample.

6. Method according to Claim 5 in which said measurements of artificial maturing are made by placing said immature sample in an inert atmosphere and subjecting it to said sequence of temperatures for said artificial maturing and then measuring a quantity of hydrocarbons released during said sequence of temperatures for said artificial maturing.

7. Method according to any one of Claims 1 to 4 in which said values representing said artificial maturing have been obtained from said reaction rate law applied to a first estimate of said kinetic parameters.

8. Method according to any one of the preceding claims in which said minimization is effected by means of a gradient method.

9. Method according to any one of the preceding claims in which there is defined on the basis of said quantity and/or quality of said hydrocarbons from said basin a scheme for operation of said basin and said sedimentary basin is exploited as a function of said quantification and/or said scheme.

10. Computer program product downloadable from a communication network and/or stored on a computer readable medium and/or adapted to be executed by a processor, comprising program code instructions which, when the program is executed by a computer, lead the latter to implement the method according to any one of the preceding claims.

**Figure 1**

Figure 2

Figure 3

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2110686 A
- US 8150669 B
- EP 2816377 A
- US 20140377872 A
- EP 3075947 A
- US 20160290107 A
- EP 3182176 A
- US 20170177764 A
- WO 2014040622 A1

- EP 0691540 B1
- US 5843787 A
- FR 3021749
- US 20150346179 A
- FR 2930350 A
- US 20090265152 A
- FR 2996038
- EP 2342557 A
- US 8796035 B

**Littérature non-brevet citée dans la description**

- **JOHN G.STAINFORTH.** Practical kinetic modeling of petroleum generation and expulsion. *Marine and Petroleum Geology,* Avril 2009, vol. 26 (4), 552-572
- **PEPPER ; CORVI.** Simple Kinetic models of petroleum formation. *Marine and Petroleum Geology,* 1995, vol. 12 (3), 291-319
- **UNGERER.** State of the art of research in kinetic modelling of oil formation and expulsion. *Org. Geochem.,* 1990, vol. 16 (1-3), 1-25
- **MCNAB, J.G. ; SMITH, P.V., JR ; BETTS, R L.** The évolution of petroleum. *Ind. Engin. Chem.,* 1952, vol. 44, 2556
- **PITT, G.J.** The kinetics of the évolution of volatile products form coal. *4th International Conférence on Coal Science,* 30 Mai 1961, 2563
- **LOUIS, M.C. ; TISSOT, B. P.** Influence de la température et de la pression sur la formation des hydrocarbures dans les argiles à kérogène. *Proceedings, 7th World Petroleum Congress,* 1967, vol. 2, 47-60
- **PHILIPPI, G.T.** On the depth, time and mechanism of petroleum génération. *Geochim. Cosmochim. Acta,* 1965, vol. 29, 1021-1049
- **JEAN BURRUS.** *contribution à l'étude du fonctionnement des systèmes pétroliers : apport d'une modélisation bi-dimensionnelle, rapport de thèse de doctorat,* 1997
- **BEHAR, F. ; LEBLOND, C. ; SAINT-PAUL, C.** Analyse quantitative des effluents de pyrolyseen milieu ouvert et fermé. *Oil and Gas Science and Technology,* 1989, vol. 44, 387-411

- **BURNHAM, A.K. ; BRAUN, R.L. ; GREGG, H.R. ; SAMOUN, A.M.** Comparison of methods for measuring kerogen pyrolysis rates and fitting kinetic parameters. *Energy and Fuels,* 1987, vol. 1, 452
- **BURNHAM, A.K. ; BRAUN, R.L. ; SAMOUN, A.M.** Further comparison of methods for measuring kerogen pyrolysis rates and fitting kinetic parameters. *Organic Geochemistry,* 1988, vol. 13 (4-6), 839-845
- **LEWAN, M.D.** Experiments on the role of water in petroleum formation. *Geochimica et Cosmochimica Acta,* 1997, vol. 61, 3691-3723
- **LEWAN, M.D. ; RUBLE, T.E.** Comparison of petroleum génération kinetics by isothermal hydrous and non-isothermal open-system pyrolysis. *Organic Geochemistry,* 2002, vol. 33, 1457-1475
- **VANDENBROUCKE, M. ; BEHAR, F. ; RUDKIEWICZ, J.L.** Kinetic modelling of petroleum formation and cracking: Implications from the high pressure/ high temperature elgin field (u.k., northsea). *Organic Geochemistry,* 1999, vol. 30 (9), 1105-1125
- **ESPITALIÉ, J. ; LAPORTE, J.L. ; MADEC, M. ; MARQUIS, F. ; LEPLAT, P. ; PAULET, J. ; BOUTEFEU, A.** Rapid method for source rock characterization, and for détermination of their petroleum potential and degree of évolution. *Revue de l'Institut Francais du Petrole et Annales des Combustibles Liquides,* 1977, vol. 32 (1), 23-42
- **ESPITALIÉ, J. ; MAKADI, K. S. ; TRICHET, J.** Role of the minerai matrix during kerogen pyrolysis. *Org. Geochem.,* 1984, vol. 6, 365-382